Europäisches Patentamt

European Patent Office

Office européen des brevets

(19)

(11) Veröffentlichungsnummer: **0 174 477**
**B1**

(12) **EUROPÄISCHE PATENTSCHRIFT**

(45) Veröffentlichungstag der Patentschrift:
**30.03.88**

(21) Anmeldenummer: **85109427.6**

(22) Anmeldetag: **23.07.85**

(51) Int. Cl.⁴: **C 12 Q 1/18, C 12 Q 1/04,
C 12 Q 1/34, C 12 Q 1/54**

(54) **Mittel und Verfahren zum Nachweis antimikrobiell wirksamer Substanzen.**

(30) Priorität: **14.08.84 DE 3429823**

(43) Veröffentlichungstag der Anmeldung:
**19.03.86 Patentblatt 86/12**

(45) Bekanntmachung des Hinweises auf die Patenterteilung:
**30.03.88 Patentblatt 88/13**

(84) Benannte Vertragsstaaten:
**BE CH DE FR GB IT LI NL SE**

(56) Entgegenhaltungen:
**EP - A - 0 054 001
EP - A - 0 091 837
EP - A - 0 122 148
DE - A - 3 327 839
US - A - 3 509 026**

(73) Patentinhaber: **Merck Patent Gesellschaft mit
beschränkter Haftung, Frankfurter Strasse 250,
D-6100 Darmstadt (DE)**

(72) Erfinder: **Kappner, Manfred, Dr., Lärchenweg 11,
D-6110 Dieburg (DE)**
Erfinder: **Metz, Harald, Dr., Am Landbach 8,
D-6101 Bickenbach (DE)**

**Beschreibung**

Die Erfindung betrifft Mittel und Verfahren zum einfachen und schnellen Nachweis von antimikrobiell wirksamen Substanzen in flüssigen Proben oder in in geeigneten Flüssigkeiten suspendierten festen Proben.

Die Bedeutung von Antibiotikarückständen in Lebensmitteln tierischer und pflanzlicher Herkunft liegt in der Verursachung von wirtschaftlichen Schäden bei der technologischen Verarbeitung bzw. im potentiellen gesundheitlichen Risiko für den Konsumenten. Die Schäden bei der Verarbeitung Antibiotika enthaltender Lebensmittel, z.B. bei der Sauermilch-, Joghurt-, Käseproduktion oder auch bei der Rohwurstherstellung beruhen darauf, dass die Aktivität der für die Herstellung fermentierter Produkte essentiellen Mikroorganismen gehemmt wird, so dass Fehlproduktionen oder Produkte mit deutlichen Qualitätsmängeln entstehen. Der regelmässige Genuss Antibiotika enthaltender Nahrungsmittel birgt für die menschliche Gesundheit zahlreiche Gefahren, z.B. toxische Schädigung, Allergien, Resistenzbildung apathogener und pathogener Darmbakterien.

Für die medizinische Diagnostik sind Informationen über Antibiotikaspiegel in Körperflüssigkeiten, wie Urin, Liquor oder Blut, von grosser Bedeutung zur verlässlichen Befunderstellung und Therapiesteuerung. Unter Chemotherapie sind die Antibiotikawirkstoffspiegel im Urin, verglichen mit den Gewebespiegeln, häufig sehr hoch. Eine mikrobiologische Keimzahluntersuchung des Urins ist deshalb unbedingt durch eine Prüfung auf antimikrobielle Aktivität zu ergänzen.

Der Nachweis von antimikrobiellen Substanzen kann nach verschiedenen physikalischen, chemischen, biochemischen und mikrobiologischen Verfahren erfolgen. Der chemische und physikalische Nachweis bestimmter mikrobieller Hemmstoffe erfolgt über die Identifizierung einer charakteristischen Komponente, wie z.B. der Guanidinogruppe im Streptomycin, des Chlors im Chloramphenicol oder durch Feststellung bestimmter Absorptionsmaxima. Immunologische Methoden (Radioimmunassay, Enzymimmunassay) eignen sich ebenfalls zum gezielten Nachweis bestimmter Antibiotika. Für Reihenuntersuchungen sind die chemischen, physikalischen und immunologischen Methoden jedoch meist ungeeignet, weil sie in einem Untersuchungsgang nur den Nachweis einer chemisch definierten Verbindung oder einer bestimmten Gruppe von chemisch definierten Verbindungen ermöglichen.

Mikrobiologische Testverfahren dagegen ermöglichen je nach Hemmstoffempfindlichkeit des Testorganismus eine Erfassung vieler antimikrobieller Substanzen. Eine einfache und rasche Identifizierung der antimikrobiellen Substanzen ist jedoch mit Ausnahme β-Lactamase-empfindlicher β-Lactam-antibiotika bisher nicht möglich.

Das Prinzip des mikrobiologischen Hemmstofftests beruht darauf, dass ein hemmstoffempfindlicher Mikroorganismus parallel in einem hemmstofffreien und potentiell hemmstoffhaltigen Nährmedium gezüchtet wird und dass irgendeine Lebensäusserung des Keimes gemessen wird; eine auftretende Differenz in den beiden Proben deutet auf die Anwesenheit von Hemmstoffen hin. Die wichtigsten in der Praxis verwendeten Testkeime sind Bacillus subtilis, Bacillus stearothermophilus, Bacillus stearothermophilus var. calidolactis, Sarcina lutea sowie Streptococcus thermophilus.

Mit einem einzigen Testorganismus, z.B. Bacillus subtilis, sind nicht alle antibakteriell wirksamen Substanzen gleich gut nachweisbar, da die Empfindlichkeit der Testorganismen gegenüber den verschiedenen Antibiotika unterschiedlich ist. Mit den erwähnten Bacillusarten kann ein Grossteil der therapeutisch interessanten Antibiotika gut nachgewiesen werden. Zum Nachweis bestimmter Antibiotika ist der am besten geeignete Testorganismus anhand der Antibiotikaempfindlichkeit auszuwählen. Für spezielle Problemstellungen, wie den Nachweis von Antibiotika in Rohstoffen für Fermentationsprozesse, ist es zur Vermeidung von Fehlchargen sinnvoll, den Rückstandstest direkt mit den Produktionsstämmen durchzuführen (z.B. Joghurt-Bakterien bei der Joghurt-Produktion).

Die Fähigkeit zur Endosporenbildung sowie die hohe Empfindlichkeit der oben erwähnten Bacillusarten gegenüber antimikrobiell wirksamen Substanzen während der Auskeimphase bzw. vegetativen Phase macht diese Bakterien zu den bevorzugten Testorganismen.

Gebräuchliche mikrobiologische Testverfahren sind z.B. Agardiffusionstests, turbidimetrische Methoden, Bestimmung von pH- oder potentiometrischen Änderungen oder radiometrische Methoden. Beim Agardiffusionsverfahren bringt man Proben des hemmstoffverdächtigen Materials auf einen festen, mit dem Testkeim beimpften Agarnährboden aus. Vorhandene Hemmstoffe diffundieren von der festen Originalprobe (z.B. Fleischstückchen) bzw. von dem mit der flüssigen Probe (z.B. Milch, Urin) getränkten Filterpapierblättchen in den Agar und verursachen auf dem nach Bebrütung dicht bewachsenen Nährboden organismenfreie «Hemmhöfe». Optimierte Agardiffusionstests mit Bacillus stearothermophilus var. calidolactis als Testorganismus benötigten Analysenzeiten von lediglich 2 - 4 Stunden. Die Haltbarkeit der gebrauchsfertigen Testplatten beträgt jedoch selbst unter optimalen Lagerungsbedingungen nur wenige Wochen; die Herstellung der Testplatten ist sehr zeitaufwendig und damit kostenintensiv.

Bei den turbidimetrischen Methoden wird das Wachstum des Testorganismus in einem hemmstofffreien und potentiell hemmstoffhaltigen Nährmedium anhand der durch das Wachstum des Testorganismus bedingten Trübungsänderung gemessen. Radiometrische Verfahren zur Hemmstoffprüfung basieren auf einer Wachstumsprüfung der Testkeime in einem Nährmedium mit C-14-markierter Glucose.

Ein Schnelltestverfahren zum Nachweis von β-Lactam-Antibiotika in einer Probe beruht z.B. auf der Hemmung der Enzymaktivität der D-Alanyl-D-alanin-carboxypeptidase durch stöchiometrische Komplexbildung mit β-Lactam-Antibiotika (EP-OS 85 667).

Die direkte Bestimmung von Enzymaktivitäten von Mikroorganismen mit Hilfe chromogener oder fluoro-

gener Substrate zur Empfindlichkeitsprüfung gegen Antibiotika ist z.B. aus EP-OS 91 837, EP-OS 54 001 und US 3 509 026 bekannt. Auch ist z.B. aus DE-OS 2 930 394 bekannt, dass Antibiotika durch Messung der intrazellulären ATP-Konzentration der Testorganismen nach bestimmten Inkubationszeiten erfasst werden können.

Der Erfindung liegt die Aufgabe zugrunde, ein Verfahren und ein Testsystem auf antimikrobiell wirksame Substanzen zur Verfügung zu stellen, das die folgenden Anforderungen erfüllt:

ein zuverlässiges Analysenergebnis in wenigen Stunden,

eine sichere Einsatzmöglichkeit auch bei schwach keimhaltigen Proben ohne vorherige Sterilisation der Proben,

eine Unempfindlichkeit des Testsystems gegenüber nicht antimikrobiell wirksamen Substanzen in der Probe,

eine gute Nachweisempfindlichkeit für die Mehrzahl der in der Praxis eingesetzten Antibiotika,

eine Testdurchführung ohne grossen Arbeits- und Materialaufwand,

die Eignung für Reihenuntersuchungen,

eine Haltbarkeit des gebrauchsfertigen Testsystems von mehreren Monaten,

eine preisgünstige kommerzielle Herstellung und die Möglichkeit zur einfachen Automatisierung.

Überraschenderweise hat sich gezeigt, dass das erfindungsgemässe Mittel und Verfahren zum Nachweis antimikrobiell wirksamer Substanzen im Gegensatz zu den bekannten Verfahren alle oben aufgeführten Anforderungen erfüllt.

Gegenstand der Erfindung ist ein Mittel zum Nachweis von antimikrobiell wirksamen Substanzen in einer Probe, bestehend aus Mikroorganismensporen, einem Nährmedium, einem chromogenen oder fluorogenen Enzymsubstrat für Hydrolasen, einem Puffersystem und gegebenenfalls einem Detergenz.

Ein weiterer Gegenstand der Erfindung ist ein Verfahren zum Nachweis von antimikrobiell wirksamen Substanzen in einer Probe, das dadurch gekennzeichnet ist, dass man ein aus Mikroorganismensporen, einem Nährmedium, einem chromogenen oder fluorogenen Enzymsubstrat für Hydrolasen, einem Puffersystem und gegebenenfalls einem Detergenz bestehendes Testsystem mit der Probe vereinigt, inkubiert und das entstandene Chromophor oder Fluorophor visuell oder photometrisch bestimmt.

Das erfindungsgemässe Mittel und Verfahren ermöglicht eine Prüfung von Proben auf antimikrobiell wirksame Substanzen — je nach Art des Testkeims — in längstens 6 Stunden durch den direkten und spezifischen Nachweis bestimmter Hydrolaseaktivitäten der Hemmstoffempfindlichen Testorganismen gegenüber chromogenen oder fluorogenen Enzymsubstraten.

Als chromogene oder fluorogene Enzymsubstrate für Hydrolasen kommen Glycoside, Carbonsäureester und Peptid-Derivate infrage, die ein Chromophor oder Fluorophor im Molekül enthalten, das unter den Bedingungen des erfindungsgemässen Verfahrens abgespalten werden kann. Bevorzugte Fluorogene sind z.B. die aus dem Stand der Technik bekannten Derivate von 4-Methyl-umbelliferon, 7-Amino-4-methyl-cumarin, Fluorescein, α- und β-Naphthol, α- und β-Naphthylamin. Bevorzugte Chromogene sind z.B. die Derivate von Nitroanilin, Nitrophenol, diazotierte Naphthole oder Naphthylamine und Derivate des Indigo.

Die chromogenen oder fluorogenen Enzymsubstrate werden in Konzentrationen von $10^{-1}$ bis $10^{-5}$ M, vorzugsweise von $10^{-2}$ bis $10^{-3}$ M, bezogen auf die Endkonzentration im Bebrütungsansatz, eingesetzt.

Bei den Hydrolasen, die die enzymatische Abspaltung des Chromophors oder Fluorophors bewirken, und die zum Nachweis der Stoffwechselaktivität der Testorganismen dienen, handelt es sich um Esterasen für aliphatische Carbonsäureester ($C_2$ - $C_{26}$), Glycosidasen wie α-D-Glucosidase, β-D-Glucosidase, α-D-Galactosidase, β-D-Xylosidase sowie um Aminopeptidasen wie D-Alanin-Aminopeptidase, L-Pyroglutaminsäure-Aminopeptidase, Glycin-Aminopeptidase oder L-Arginin-Aminopeptidase.

Die für den Hemmstofftest vorzugsweise eingesetzten Bacillusarten zeichnen sich durch sehr gute Esteraseaktivitäten für aliphatische Carbonsäureester mit beispielsweise 4-Methylumbelliferon als Fluorophor aus.

Bacillus subtilis zeigt z.B. hervorragende Esteraseaktivitäten für folgende fluorogene Substrate: 4-Methylumbelliferyl-acetat, Fluorescein-di-acetat, 4-Methylumbelliferyl-propionat, 4-Methylumbelliferyl-butyrat, 4-Methylumbelliferyl-i-butyrat, 4-Methylumbelliferyl-valerianat, 4-Methylumbelliferyl-i-valerianat, 4-Methylumbelliferyl-trimethylacetat, 4-Methylumbelliferyl-caproat, 4-Methylumbelliferylheptanoat, 4-Methylumbelliferyl-caprylat, 4-Methylumbelliferyl-nonanoat, 4-Methylumbelliferyl-caprinat, 4-Methylumbelliferyl-laurat, 4-Methylumbelliferyl-myristat, 4-Methylumbelliferyl-palmitat, 4-Methylumbelliferyl-oleat, 4-Methylumbelliferyl-elaidat, 4-Methylumbelliferyl-stearat, 4-Methylumbelliferyl-lignocerat.

Während die Mehrheit der Esteraseaktivitäten für die verschiedenen Substrate nicht spezifisch nur bei den erwähnten Bacillus-Testorganismen vorkommt — die Testorganismen zeichnen sich lediglich durch besonders hohe Enzymaktivitäten aus — besitzen einige Glycosidasen, vor allem die α-D-Glucosidase, eine hohe Spezifität für Bacillus subtilis, Bacillus stearothermophilus sowie Bacillus stearothermophilus var. calidolactis. Bei der Mehrzahl der gramnegativen und grampositiven Bakterien können unter den Testbedingungen keine oder nur geringe α-D-Glucosidase-Aktivitäten nachgewiesen werden. Die α-D-Glucosidase eignet sich somit in Hemmstofftests gut zum Nachweis der Stoffwechselaktivitäten von Bacillus subtilis, Bacillus stearothermophilus oder Bacillus stearothermophilus var. calidolactis, auch in Proben, die hemmstoffresistente Bakterien enthalten, Bacillus subtilis besitzt weiterhin gute β-D-Glucosidase- sowie α-D-Galactosidase-Aktivitäten. Bacillus stearothermophilus zeigt ebenfalls eine gute α-D-Galactosidase-Aktivität.

Enthält das erfindungsgemässe Mittel Sporen von Bacillus subtilis, so wird als chromogenes oder fluorogenes Substrat für Glycosidasen vorzugsweise ein β-D-Glucosid oder ein α-D-Glucosid eingesetzt und für Aminopeptidasen eine D-Alanin-(Chromophor/ Fluorophor)-Verbindung oder ein D-Alanin-(Chromophor/Fluorophor) enthaltendes Peptid. Enthält das erfindungsgemässe Mittel Sporen von Bacillus stearothermophilus, so wird als chromogenes oder fluorogenes Substrat vorzugsweise ein α-D-Glucosid oder ein α-D-Galactosid eingesetzt.

Als Substrat für die α-D-Glucosidase kann beispielsweise p-Nitrophenyl-α-D-glucosid, 4-Methylumbelliferyl-α-D-glucosid, Fluorescein-α-D-glucosid, α- bzw. β-Naphthyl-α-D-glucosid, Indoxyl-α-D-glucosid, 5-Brom-3-indoxyl-α-D-glucosid, 5-Brom-4-chlor-3-indoxyl-α-D-glucosid und Indoxyl-α-D-glucosid-pentaacetat verwendet werden.

Bacillus subtilis zeigt unter den Testbedingungen eine deutliche und organismenspezifische D-Alanin-Aminopeptidase-Aktivität bei D-Alanin-p-amido-4-methylcumarin als fluorogenem Substrat bzw. D-Alanin-p-nitroanilin als chromogenem Substrat. Weitere geeignete Substrate sind Peptid-Derivate, die am N-terminalen Ende der Verbindung D-Alanin-(Chromophor/Fluorophor) weitere Aminosäuren wie Glycin, Alanin, Valin, Leucin, Isoleucin, Asparaginsäure, Glutaminsäure usw. enthalten, z.B. Glycyl-D-alanin-7-amido-4-methyl-cumarin.

Als Testorganismen für antimikrobiell wirksame Substanzen werden vorzugsweise Sporen von Bacillus subtilis, Bacillus stearothermophilus oder Bacillus stearothermophilus var. calidolactis in einer Konzentration von $10^4$ - $10^9$ Sporen pro Inkubationsansatz verwendet. Alternativ zu den Bakteriensporen zum Nachweis von antibakteriell wirksamen Substanzen können auch Pilzsporen zum Nachweis von Antimycotika eingesetzt werden. Geeignete Testorganismen für antimykotische Substanzen sind bei den Dermatophyten z.B. Trichophyton rubrum und Trichophyton schoenleinii, bei den pathogenen Hefen Candida albicans und Torulopsis glabrata. Für den allgemeinen Nachweis von Antimykotika können Aspergillus oder Penicillium-Arten verwendet werden.

Als Nährmedium kann z.B. MUELLER-HINTON-Bouillon, Hirn-Herz-Bouillon, Caseinpepton-Sojamehlpepton-Bouillon in geeigneten Puffern wie Phosphatpuffer, Tris/HCl-Puffer oder anderen gebräuchlichen Puffersystemen eingesetzt werden. Ein bevorzugter Puffer ist 0,1 M Kaliumphosphatpuffer. Die Wahl des Puffers sowie des pH-Wertes richtet sich sowohl nach den optimalen Testbedingungen des nachzuweisenden Enzyms als auch nach den geeignetsten physiologischen Bedingungen für die Testorganismen. Der Fachmann kann die geeigneten Bedingungen nach Routinemethoden ermitteln.

Durch Zusatz eines Detergenz zum Testansatz in Konzentrationen, die keinen negativen Effekt auf das Wachstum der Bakterien besitzen, kann bei Antibiotika mit Primärwirkort in der Mureinbiosynthese die Nachweisempfindlichkeit verbessert werden. Zellen mit gestörter Mureinbiosynthese platzen in Gegenwart von Detergenz und bilden keine large-Formen, die bei der Enzymaktivitätsbestimmung ein normales ungehemmtes Wachstum der Testorganismen vortäuschen. Detergenzien werden vorzugsweise in Konzentrationen von $10^{-4}$ - $10^{-6}$ M, bezogen auf die beabsichtigte Endkonzentration, zugesetzt. Vorzugsweise enthält das Mittel nach der Erfindung kationenaktive, anionenaktive und/oder nichtionogene Detergenzien wie Polyoxyethylenderivate der Sorbitanester wie Polyoxyethylensorbitanmonolaurat, -sorbitanmonopalmitat und -sorbitanmonooleat.

Glucosekonzentrationen in Testansätzen von mehr als 1 g/l führen teilweise zur Reprimierung der Glycosidasen. Durch Oxidation der Glucose kann dieser «Glucoseeffekt» aufgehoben werden.

Aus der Vielzahl der antimikrobiell wirksamen Substanzen, die mit dem erfindungsgemässen Mittel und Verfahren nachgewiesen werden können, seien z.B. die folgenden genannten: Aminoglycosid-Antibiotika wie Streptomycin, Neomycin, Kanamycin, Gentamycin, Spectinomycin; Chloramphenicol und -Derivate wie Thiamphenicol; Makrolid-Antibiotika wie Erythromycin, Leucomycin, Oleandomycin, Spiramycin, Carbomycin; Lincomycine wie Clindamycin, Lincomycin; Penicilline wie Phenoxymethylpenicillin, Ampicillin, Penicillin G, Penicillin V, Dicloxacillin-Natrium, Furoxacillin, Mecillinam; Cephalosporine wie Cefalothin, Cefamandol, Cefazedon, Cefazolin, Cefoxitin, Cefuroxim; Peptid-Antibiotika wie Bacitracin, Gramicidin, Polymyxine; Rifamycine wie Rifampicin, Rifamycin; Steroid-Antibiotika wie Fusidinsäure; Tetracycline wie Doxycyclin, Minocyclin, Chlortetracyclin, Oxytetracyclin; sowie sonstige Antibiotika z.B. Cycloserin, Fosfomycin, Vancomycin, Sulfonamide, Nitrafurantoin, Nalidixinsäure.

Die Nachweisempfindlichkeit für die verschiedenen antimikrobiell wirksamen Substanzen ist von der Hemmstoffempfindlichkeit der verwendeten Testorganismen, der Anzahl der Testorganismen im Inkubationsansatz sowie der Nährmediumzusammensetzung abhängig und entspricht mindestens derjenigen der bekannten Hemmstofftests. Der Arbeitsaufwand zur Durchführung des Tests auf antimikrobiell wirksame Substanzen ist bei dem erfindungsgemässen Verfahren auf ein Minimum reduziert. Da die Komponenten des gebrauchsfertigen Testansatzes in getrockneter Form vorliegen, ist eine ausreichende Haltbarkeit von mindestens mehreren Monaten gewährleistet.

Die Durchführung des Tests auf antimikrobiell wirksame Substanzen ist sehr einfach: die flüssige Probe oder die in einer geeigneten Flüssigkeit suspendierte feste Probe wird in ein Reaktionsfeld des gebrauchsfertigen Testansatzes gegeben, anschliessend wird das Testsystem bei der optimalen Wachstumstemperatur des Testorganismus bebrütet. Bei Bacillus subtilis-Sporen als Testorganismen beispielsweise ist in Abhängigkeit von der Auskeimungsgeschwindigkeit der Sporen meist nach einer Bebrütungszeit von 4 Stunden bei der optimalen Wachstumstemperatur des Testorganismus eine sichere Aussage über das Vorliegen von antimikrobiell wirksamen Substanzen in der Probe durch Bestimmung der Konzentration des hydrolytisch gespaltenen Anteils des chromogenen bzw. fluorogenen Enzymsubstrats möglich. Der Test kann zuverlässig mit blossem Auge durch Vergleich der Farbintensität des Chromophors oder der Fluoreszenzintensität des Fluorophors im Testansatz mit Standards ausgewertet werden. Bei Konzentrationen von antimikrobiell wirksamen Substanzen, die grösser oder gleich der minimalen Hemmkonzentration sind, werden im Vergleich zur hemmstofffreien Probe keine oder nur minimale Konzentrationen des Enzymsubstrates hydrolytisch gespalten.

Der gebrauchsfertige, in trockener Form vorliegende Testansatz des Testsystems hat folgende Zusammensetzung: Keime des Testorganismus (vorz. Sporen), ein Nährmedium, ein chromogenes oder fluorogenes Enzymsubstrat für Hydrolasen, ein geeignetes Puffersystem und ggf. ein Detergenz. Der Testansatz kann jedoch auch Bestandteile zur Beseitigung störender Substanzen enthalten, z.B. ein Enzym zur Oxidation von Glucose.

Das Testsystem besteht wahlweise aus einem Tiefziehteil bzw. einem Spritzgussteil mit zahlreichen, 0,5 bis 1 ml grossen Kavitäten oder aus einer handelsüblichen Mikrotiterplatte. Zur Vermeidung von Flüssigkeitsverlust bei der Bebrütung können die Kavitäten des Testsystems mit einer Haft- oder Klebefolie bzw. einem Deckel verschlossen werden. Die Komponenten des gebrauchsfertigen Testansatzes, die in getrockneter Form vorliegen, können in den Kavitäten auch auf Trägern mit einer guten Aufnahmefähigkeit für Flüssigkeiten aufgebracht sein (z.B. Filterpapierblättchen). Alternativ können die Komponenten des gebrauchsfertigen Testansatzes auch auf Reaktionszonen von Teststäbchen mit einer guten Aufnahmefähigkeit von Flüssigkeiten aufgebracht werden. Die Probe kann entweder auf eine oder mehrere Reaktionszonen des Teststreifens, die eine definierte Aufnahmefähigkeit für Flüssigkeiten besitzen, nach dem Eintauchverfahren aufgebracht werden, oder es wird eine definierte Probenmenge mit einer Pipette auf die Reaktionszone aufgetragen. Die Zusammensetzung des gebrauchsfertigen Testansatzes kann auf einzelnen Reaktionszonen verschieden sein; es können z.B. pH-Wert, Puffersystem, Enzymsubstrate, Testorganismen, Nährmedium sowie Art und Konzentration des Detergenz variiert werden.

Die Reaktionszone auf dem Teststreifen kann z.B. aus speziellen Filterpapieren und/oder einer polymeren Matrix aus z.B. Carboxymethylcellulose, Calciumalginat, Polyvinylalkoholen, Polyvinylpyrrolidonen bestehen. Die Testorganismen können auch trägerfixiert vorliegen. Zur Verhinderung des Austrocknens bei der Bebrütung werden die Teststreifen in entsprechenden, in der Form angepassten und dicht verschliessbaren Bebrütungsgefässen aufbewahrt.

Die Erfindung wird durch die folgenden Beispiele näher erläutert.

*Beispiel 1*

*Hemmstofftest unter Nachweis der α-D-Glucosidase von Bacillus stearothermophilus*

Herstellung des gebrauchsfertigen Testsystems

50 µl einer 10fach konzentrierten MUELLER-HINTON-Bouillon in 0,1 M Kalium-Phosphatpuffer, pH 7,5, 50 µl einer $10^{-2}$ M wässrigen Lösung von p-Nitrophenyl-α-D-glucosid sowie 10 µl einer $5 \cdot 10^{-4}$ M Lösung von Polyethylenglycol-mono[p-(1.1.3.3-tetramethylbutyl)-phenyl]-ether werden in eine 1-ml-Polystyrolküvette gegeben. Die Lösung wird anschliessend gefriergetrocknet. Das Lyophilisat wird mit 10 µl einer wässrigen Sporensuspension von Bacillus stearothermophilus (KUNDRAT) mit $5 \cdot 10^{7}$ Sporen/ml versetzt und anschliessend erneut lyophilisiert. Nach der Lyophilisation wird die gebrauchsfertige Testküvette mit einem Deckel verschlossen, in einem Beutel aus aluminiumkaschierter Polyethylenfolie eingeschweisst und bis zum Gebrauch bei 4 - 6°C kühl gelagert.

Durchführung des Hemmstofftests

Jeweils 0,5 ml einer wässrigen Probenlösung mit 0/0,5/0,1/0,05/0,01/0,005/0,001/0,0005 µg/ml Phenoxymethylpenicillin werden in je eine gebrauchsfertige Testküvette gegeben.

Die Küvetten werden verschlossen und 4 Stunden bei 60°C bebrütet. Anschliessend wird nach Zugabe von 50 µl 1 N Natronlauge die Extinktion des Testansatzes bei 390 nm bestimmt.

Die Probe ohne Phenoxymethylpenicillin bzw. mit 0,001 sowie 0,0005 µg/ml Phenoxymethylpenicillin zeigt eine deutlich höhere Extinktion bei 390 nm als die Proben mit 0,005 µg/ml oder einer höheren Phenoxymethylpenicillin-Konzentration.

Die Nachweisgrenze von Phenoxymethylpenicillin beträgt somit unter den gewählten Testbedingungen 0,005 µg/ml.

*Beispiel 2*

*Hemmstofftest unter Nachweis der β-D-Glucosidase von Bacillus subtilis*

Herstellung des gebrauchsfertigen Testsystems

50 µl Caseinpepton-Sojamehlpepton-Bouillon in 0,1 M phosphatpuffer, pH 7,5, werden auf Filterpapierblättchen (Durchmesser 9 mm) gegeben. Die Testblättchen werden dann bei 40°C getrocknet. Anschliessend werden 50 µl einer $1 \cdot 10^{-2}$ M-Lösung von p-Nitrophenyl-β-D-glucosid in Ethanol auf die Testblättchen pipettiert und bei 40°C trocknen gelassen. Auf die getrockneten Testblättchen gibt man 10 µl einer wässrigen Sporensuspension von Bacillus subtilis mit $10^{7}$ Sporen/ml. Die Testblättchen werden anschliessend erneut bei 40°C getrocknet und einzeln in 2-ml-Schraubglasröhrchen verpackt. Die gebrauchsfertigen Testblättchen werden bis zum Gebrauch bei 4 - 6°C gelagert.

Durchführung des Hemmstofftests

Milchproben werden mit Chloramphenicol zu Konzentrationen von 32/16/8/4/2/1/0,5/0,25 µg/ml versetzt. Jeweils ein gebrauchsfertiges Testblättchen wird in die einzelnen Milchproben eingetaucht und anschliessend wieder in das Schraubglasröhrchen zurückgelegt. Nach 4 Stunden Bebrütung der dicht verschlossenen Schraubglasröhrchen bei 37°C werden auf die einzelnen Testblättchen 20 µl 1 N Natronlauge gegeben.

Das Testblättchen ohne Chloramphenicol in der Milchprobe sowie diejenigen mit 0,25 bzw. 0,5 µl/ml zeigen eine intensive gelbe Farbe im Gegensatz zu den Testblättchen, die in Milchproben mit Chloramphenicol-Konzentrationen von 2 µg/ml und mehr eingetaucht wurden. Das Testblättchen mit 1,0 µg/ml Chloramphenicol zeigt eine schwache Gelbfärbung.

Die Nachweisgrenze für Chloramphenicol beträgt somit unter den Testbedingungen 1 - 2 µg/ml.

*Beispiel 3*

*Hemmstofftest unter Nachweis der Butyrat-Esterase von Bacillus subtilis.*

Herstellung des gebrauchsfertigen Testsystems

50 µl einer MUELLER-HINTON-Bouillon in 0,1 M Phosphatpuffer, pH 6,5, sowie 25 µl einer $2 \times 10^{-2}$ M Lösung von 4-Methylumbelliferyl-butyrat in Aceton werden auf Filterpapierblättchen (Durchmesser 9 mm) gegeben. Die Testblättchen werden anschliessend bei 40°C in einem Warmluftstrom getrocknet. Auf das getrocknete Testblättchen gibt man 10 µl einer wässrigen Sporensuspension von Bacillus subtilis mit $10^7$ Sporen/ml. Die Testblättchen werden anschliessend erneut bei 40°C in einem Warmluftstrom getrocknet und bis zum Gebrauch in einem 2-ml-Schraubglasröhrchen bei 4 bis 6°C aufbewahrt.

Durchführung des Hemmstofftests

Die Testblättchen werden in 0,1 M Phosphatpuffer, pH 6,5, mit verschiedenen Konzentrationen an Tetracyclin (16/8/4/2/1/0,5/0,25/0,1/0,05/0,025/0,01 µg/ml) eingetaucht und anschliessend wieder in das Schraubglasröhrchen zurückgelegt. Nach 4 Stunden Bebrütung der dicht verschlossenen Schraubglasröhrchen bei 37°C wird die Fluoreszenz der Testblättchen durch Bestrahlung mit einer Wellenlänge von 366 nm untersucht.

Das Testblättchen ohne Tetracyclin in der Probe sowie diejenigen mit 0,01/0,25/0,05 µg/ml Tetracyclin in der Probe zeigen eine intensive blaue Fluoreszenz im Gegensatz zu den Testblättchen mit 0,1 µl/ml Tetracyclin in der Probe bzw. höheren Tetracyclin-Konzentrationen. Die Nachweisgrenze für Tetracyclin beträgt somit unter den Testbedingungen 0,1 µg/ml.

**Patentansprüche**

1. Mittel zum Nachweis von antimikrobiell wirksamen Substanzen in einer Probe, bestehend aus Mikroorganismensporen, einem Nährmedium, einem chromogenen oder fluorogenen Enzymsubstrat für Hydrolasen, einem Puffersystem und gegebenenfalls einem Detergenz.

2. Mittel nach Anspruch 1, dadurch gekennzeichnet, dass das chromogene oder fluorogene Enzymsubstrat ein Glycosid, ein Carbonsäureester oder ein Peptid-Derivat ist.

3. Mittel nach den Ansprüchen 1 und 2, dadurch gekennzeichnet, dass es Sporen von Bacillus subtilis und als chromogenes oder fluorogenes Substrat für Glycosidasen ein β-D-Glucosid oder ein α-D-Glucosid enthält.

4. Mittel nach den Ansprüchen 1 bis 3, dadurch gekennzeichnet, dass es Sporen von Bacillus stearothermophilus und als chromogenes oder fluorogenes Substrat für Glycosidasen ein α-D-Glucosid oder ein α-D-Galactosid enthält.

5. Mittel nach den Ansprüchen 1 bis 4, dadurch gekennzeichnet, dass es Sporen von Bacillus subtilis und als chromogenes oder fluorogenes Substrat für Aminopeptidasen eine D-Alanin-(Chromophor/Fluorophor)-Verbindung oder ein D-Alanin-(Chromophor/Fluorophor) enthaltendes Peptid enthält.

6. Mittel nach den Ansprüchen 1 bis 5, dadurch gekennzeichnet, dass es in getrockneter oder lyophilisierter Form vorliegt.

7. Mittel nach den Ansprüchen 1 bis 6, dadurch gekennzeichnet, dass es in einer polymeren Matrix vorliegt.

8. Verfahren zum Nachweis von antimikrobiell wirksamen Substanzen in einer Probe, dadurch gekennzeichnet, dass man ein aus Mikroorganismensporen, einem Nährmedium, einem chromogenen oder fluorogenen Enzymsubstrat für Hydrolasen, einem Puffersystem und gegebenenfalls einem Detergenz bestehendes Testsystem mit der Probe vereinigt, inkubiert und das freigesetzte Chromophor oder Fluorophor visuell oder photometrisch bestimmt.

**Claims**

1. Reagent for detecting antimicrobially active substance in a sample, consisting of spores of a microorganism, a nutrient medium, a chromogenic or fluorogenic enzyme substrate for hydrolase, a buffer system and, if appropriate, a detergent.

2. Reagent according to claim 1, characterised in that the chromogenic or fluorogenic enzyme substrate is a glycoside, a carboxylic acid ester or a peptide derivative.

3. Reagent according to claim 1 and 2, characterised in that it contains spores of Bacillus subtilis and, as the chromogenic or fluorogenic substrate for glycosidases, a β-D-glucoside or an α-D-glucoside.

4. Reagent according to claims 1 to 3, characterised in that it contains spores of Bacillus stearothermophilus and, as a chromogenic or fluorogenic substrate for glycosidases, an α-D-glucoside or an α-D-galactoside.

5. Reagent according to claims 1 to 4, characterised in that it contains spores of Bacillus subtilis and, as a chromogenic or fluorogenic substrate of amino-peptidases, a D-alanine (chromophore/fluorophore) compound or a peptide containing a D-alanine (chromophore/fluorophore).

6. Reagent according to claims 1 to 5, characterised in that it is present in a dried or liophilised form.

7. Reagent according to claims 1 to 6, characterised in that it is present in a polymeric matrix.

8. Method for detecting antimicrobially active substances in a sample, characterised in that a test system consisting of spores of a microorganism, a nutrient medium, a chromogenic or fluorogenic enzyme substrate for hydrolases, a buffer system and, if appropriate, a detergent is combined with the sample and incubated, and the released chromophore or fluorophore is determined visually or photometrically.

## Revendications

1. Moyen pour la mise en évidence de substances possédant une activité antimicrobienne, constitué par des spores de microorganismes, un milieu nutritif, un substrat enzymatique chromogène ou fluorogène pour les hydrolases, un système tampon et éventuellement un détergent.

2. Moyen selon la revendication 1, caractérisé en ce que le substrat enzymatique chromogène ou fluorogène est un glycoside, un ester d'acide carboxylique ou un dérivé peptidique.

3. Moyen selon les revendications 1 et 2, caractérisé en ce qu'il contient des pores de Bacillus subtilis et en tant que substrat chromogène ou fluorogène pour les glycosidases un bêta-D-glucoside ou un alpha-D-glucoside.

4. Moyen selon les revendications 1 à 3, caractérisé en ce qu'il contient des spores de Bacillus stearothermophilus et en tant que substrat chromogène ou fluorogène pour les glycosidases un alpha-D-glucoside ou un alpha-D-galactoside.

5. Moyen selon les revendications 1 à 4, caractérisé en ce qu'il contient des spores de Bacillus subtilis et en tant que substrat chromogène ou fluorogène pour les aminopeptidases un composé D-alanine-(chromophore/fluorophore) ou un peptide contenant D-alanine-(chromophore/fluorophore).

6. Moyen selon les revendications 1 à 5, caractérisé en ce qu'il existe sous forme séchée ou lyophilisée.

7. Moyen selon les revendications 1 à 6, caractérisé en ce qu'il existe sous forme de matrice polymère.

8. Procédé pour la mise en évidence de substances possédant une activité antimicrobienne dans un échantillon, caractérisé en ce qu'un système de test composé de spores de microorganismes, d'un milieu nutritif, d'un substrat enzymatique chromogène ou fluorogène pour les hydrolases, d'un système tampon et éventuellement d'un détergent, est combiné avec l'échantillon, incubé et le chromophore ou le fluorophore libéré est déterminé visuellement ou par photométrie.